(19) Europäisches Patentamt
European Patent Office
Office européen des brevets

(11) EP 3 811 982 A1

(12) EUROPEAN PATENT APPLICATION

(43) Date of publication:
28.04.2021 Bulletin 2021/17

(51) Int Cl.:
A61L 27/22 (2006.01)
A61L 27/56 (2006.01)
A61L 27/54 (2006.01)
C12N 5/00 (2006.01)

(21) Application number: 19306387.2

(22) Date of filing: 25.10.2019

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
• Université de Strasbourg
67000 Strasbourg (FR)
• Centre National de la Recherche Scientifique
75016 Paris (FR)
• INSERM (Institut National de la Santé et de la Recherche Médicale)
75013 Paris (FR)

(72) Inventors:
• ALOUI, Eya
67100 STRASBOURG (FR)
• DE GIORGI, Marcella
67000 STRASBOURG (FR)
• FRISCH, Benoît
67100 STRASBOURG (FR)
• LAVALLE, Philippe
67370 WINTZENHEIM-KOCHERSBERG (FR)
• SCHAAF, Pierre
67120 MOLSHEIM (FR)

(74) Representative: Novagraaf Technologies
Bâtiment O2
2, rue Sarah Bernhardt
CS90017
92665 Asnières-sur-Seine Cedex (FR)

(54) **PROTEIN-BASED BIOMATERIAL WITH VISCOELASTIC BEHAVIOUR, PROCESS FOR OBTAINING IT AND USES THEREOF**

(57) The present invention relates to a process of preparation of a biomaterial comprising the steps of:
a) Preparing a solution comprising at least one protein having a solubility in water superior or equal to about 10 mg/ml and at least one salt having solubility in water superior or equal to about 500 mg/ml,
b) Evaporating the solution obtained in step a) as is, as a foam obtained by foaming the solution obtained in step a), or as a mixture thereof, at a temperature comprised of from 20 to 50°C in atmospheric pressure or at lower temperatures under vacuum or at a pressure lower than atmospheric pressure, until the formation of two non-miscible phases or until obtaining a substantially dry solid, thereby obtaining a biomaterial.

The present invention also relates to a biomaterial obtainable by the process, and to the use of the biomaterial as a support for in vitro tissue engineering and/or for in vitro cell culture and in vitro expansion and/or as an implantable medical device, or as a drug.

Figure 5

EP 3 811 982 A1

## Description

### Technical field

The present invention refers to a process of preparation of a biomaterial, the biomaterial obtainable by the process, as well as the use of the biomaterial as a support for tissue engineering, for cell culture or expansion, as an implantable medical device and as a drug.

Therefore, the present invention has utility in medical fields, notably for tissue engineering, drug delivery, wound dressing and implants.

In the description below, the references into brackets ([]) refer to the listing of references situated at the end of the text.

### Background of the Invention

Biomaterials are widely used in various therapeutic applications such as tissue engineering, drug delivery, wound dressings and implants.

Synthetic, living or hybrid, they have gained in a few decades all therapeutic areas: cardiovascular, surgery and orthopedics, dental, ophthalmology, dermatology, urology, nephrology, neurology, endocrinology, especially to regenerate or improve the function of a tissue.

There are several types of biomaterials, but four main categories of biomaterials can be envisaged:

- Metals and metal alloys,
- Ceramics in the broad sense,
- Polymers and soft matter,
- Materials of natural origin, such as coral or other constituents extracted from plant or animal organisms, for example chitin, alginate, heparin, fucoidan, cellulose, collagen or fibrin.

This last category of biomaterial is particularly interesting, as materials of natural origin are generally naturally biocompatible, and biodegradable.

Multiple techniques involving heat-aggregation or cross-linking have been used to prepare biomaterials based on materials of natural origin. However, the use of high temperatures and cross-linking agents may lead to irreversible denaturation and alterations of the material's structure, resulting in the loss of its biological properties and possibly revealing new antigenic sites, which can trigger an inflammatory response.

Thus, a need exists of alternative biomaterials based on materials of natural origin that do not have these drawbacks, and that fulfil the needs more natural biomaterials.

### Description of the invention

By extensive researches, Applicants have developed new biomaterials exhibiting remarkable mechanical properties and a good stability in water, acidic solutions and cell culture medium.

Surprisingly, the obtained biomaterials display a solid-like behaviour, making it a good candidate as support for tissue engineering or for cell culture.

The present disclosure reports the first biomaterials possibly made entirely of a unique type of protein, or of several chosen proteins that is/are not denatured during the process of preparation of the biomaterial. This implies that the biomaterials of the invention are not likely to trigger an inflammatory response in subjects. Furthermore, the biomaterials are non-cytotoxic and are favourable to cell adhesion and colonization.

As it is made of proteins, the biomaterials of the invention are completely biodegradable.

The process of preparation developed by the Applicants makes it possible to obtain the biomaterial without any chemical or harsh/denaturant formulation conditions. It also allows to modulate the properties of the biomaterials, notably mechanical and intrinsic properties, and to obtain functionalized biomaterials with new properties. Therefore, the Applicants provide versatile biomaterials whose properties can be modulated to adapt to the requirements of targeted therapeutic applications.

Accordingly, in a first aspect, the present invention provides a process of preparation of a biomaterial comprising the steps of:

a) Preparing a solution comprising at least one protein having a solubility in water superior or equal to about 10 mg/ml, and at least one salt having solubility in water superior or equal to about 500 mg/ml,

b) Evaporating the solution obtained in step a) as is, as a foam obtained by foaming the solution obtained in step a), or as a mixture thereof, at a temperature comprised of from 20 to 50°C in atmospheric pressure or at lower temperatures under vacuum or at a pressure lower than atmospheric pressure, until the formation of two non-miscible phases or until obtaining a substantially dry solid,

thereby obtaining a biomaterial.

The process of the invention has the significant advantage not to use any covalent cross-linking or heat-aggregation step, thereby allowing obtaining a biomaterial with non-denatured proteins.

The at least one protein used in step a) may be any protein having a solubility in water that is superior or equal to about 10 mg/ml at a temperature of 20°C, for example superior or equal to 20 mg/ml, or to 40 mg/ml, or to 50 mg/ml. For example, the proteins may have a solubility comprised between about 10 mg/ml and 1000 mg/ml. The solubility in water of the protein may be measured by any method known by the person skilled in the art, for example high-performance liquid chromatography (HPLC), attenuated total reflection (ATR)-FTIR spectroscopy, Raman spectroscopy or focused beam reflectance mode (FBRM) measurement. Such proteins may be for example selected among serum proteins such as albumin or globulin. Albumin may be notably selected among human serum albumin, bovine serum albumin, porcine serum albumin, ovalbumin, vegetal albumin, and recombinant albumin, for example recombinant human albumin structurally equivalent to native human serum albumin and produced in rice. Albumin may also be an albumin nanoparticle. As indicated above, "at least one" protein may be used in the process of the invention, meaning that 1, or 2, or 3, or 4, or more different proteins may be used in the solution of step a). Preferably, it may be used between 1 and 3 different proteins. The concentration of the protein in the solution of step a) may be any concentration allowing a mixture with the salt. It can be for example comprised between 10 mg/ml and 500 mg/ml. The man skilled in the art is able to adapt the concentration of protein in the solution depending on the nature of the salt and the salt concentration. However, as explained more in details below, the molar ratio between salt and protein is more important than the protein concentration to obtain a biomaterial with desired features.

The at least one salt in step a) may be any salt having solubility in water superior or equal to about 500 mg/ml at a temperature of 20°C, for example superior or equal to 700 mg/ml, or to 900 mg/ml, or to 1200 mg/ml. For example, the salts may have a solubility comprised between about 500 mg/ml and 3000 mg/mL. The solubility in water of the salt may be measured by any method known by the person skilled in the art, for example ion chromatography, gas chromatography, acid-base titration, potentiometric titration, volumetry, weighing or Raman spectroscopy. The at least one salt may be for example selected among NaBr, NaI, KI, $CaCl_2$, $MgCl_2$, $KC_2H_3O_2$ and $NH_4HCO_2$. As indicated above, "at least one" salt may be used in the process of the invention, meaning that 1, or 2, or 3, or 4, or more different salts may be used. Preferably, it may be used between 1 and 3 salts. For example, the at least one salt may comprise NaBr and NaI, NaBr and $CaCl_2$, KI and $CaCl_2$. The concentration of the salt in the solution of step a) may be any concentration allowing a mixture with the protein. It can be for example comprised between 0.01 M and 40 M. The man skilled in the art is able to adapt the concentration of salt in the solution depending on the nature of the protein and the protein concentration. However, as explained more in details below, the molar ratio between salt and protein is more important than the salt concentration to obtain a biomaterial with desired features.

Preferably, the at least one protein and the at least one salt may be, in step a), in a molar ratio that is dependent of the nature of the protein and of the nature of the salt for obtaining the biomaterial. Since it has been demonstrated by the Applicants that the formation of the biomaterial is dependent on the paired effect of both concentrations of proteins and salt, the molar ratio salt/protein is a more relevant and reliable parameter to evaluate membrane formation. Knowing this, the molar ratio may be determined by the skilled person in view of his general knowledge and of the desired properties of the insoluble biomaterial, notably its firmness, without undue burden. For example, the molar ratio may be comprised between 100 and 3000, for example between 300 and 2500, or between 400 and 2000, or between 600 and 1500, or between 650 and 1000, depending on the salt and of the protein used in step a). For example, molar ratio for a mixture of NaBr and albumin may be of 664.

The solution of step a) may be realised by mixing the at least one protein and the at least one salt in an adapted solvent or mixture of solvents, in non-denaturing conditions. The solvent may be chosen by the skilled person in view of his general knowledge and of the nature of the salt and protein, without undue burden. For example, the solvent may be chosen among water, a buffer such as acetate buffer, a mixture of water and buffer and of another water miscible solvent such as ethanol, methanol, acetone, DMF or DMSO. The temperature of the solution during step a) may be of between 5°C and 40°C.

Step a) can be carried out at any pH avoiding the denaturation of proteins, which is known by the skilled person. Preferably, step a) is performed at a pH comprised between 3.0 and 9.0, the value of 3.0 being optionally excluded. The pH may be for example of between 4.0 and 9.0, or of between 4.0 and 8.0.

The mixture may be realised or may be transferred on any container or support adapted to receive such a mixture. It can be for example a mold of glass or silicone, microscopy or microarray substrates, cell and tissue culture dishes or microwell plates, or a polymeric support around which the biomaterial can take shape. Advantageously, the support may be chosen in view of the desired surface area, shape and thickness of the biomaterial to be obtained. In this purpose, the volume of the mixture to be poured in the container may be chosen depending on the surface area of the support and/or of the desired thickness of the biomaterial. The biomaterial may have any shape, for example a membrane, a full or hollow cylinder, a cone, a sphere, a pavement. For information, the ratio M/S, which is the ratio between the initial weight of protein used for the formulation and the area of the container as exemplified below, may be comprised between 20 mg/cm$^2$ and 400 mg/cm$^2$.

Step b) of evaporation may be performed on solution obtained in step a) as in. In this case, step b) is performed directly after step a), or after an intermediate step that does not change the nature or the physical structure of the solution obtained in step a).

Alternatively, step b) may be performed on a foam obtained by foaming the solution obtained in step a). The foam may be obtained, for example, by applying mechanical work on the solution obtained in step a) to increase the surface area of the solution. This can be performed by any method known by the skilled person, for example agitation, dispersing a large volume of gas into the solution obtained at step a), or injecting a gas into the solution obtained at step a).

In another embodiment, step b) may be performed on a mixture of the solution obtained in step a) and of the foam obtained by foaming the solution obtained in step a). In this case, a part of the solution obtained in step a) may be taken and foamed in a separate container. The resulting foam may be evaporated as is or put back with the solution and mixed with it then evaporated as in step b). Preferably, mixing is made gently in order to preserve the foam. The evaporation of the foam produces a highly porous biomaterial.

Evaporating of step b) is made in order to allow the formation of two non-miscible phases or to obtain a substantially dry solid. Advantageously, it is carried out under conditions that make possible to avoid denaturation of the proteins present in the solution, the foam or the mixture thereof. In this purpose, temperature and pressure may be determined, and adjusted relative to each other, to achieve this goal. The person skilled in the art is able to determine these parameters depending on the kind of proteins or salt, and according to his general knowledge. For example, temperature may be comprised from 20 to 50°C in atmospheric pressure, for example from 25 to 40°C, or from 25 to 35 °C, or from 20 to 30°C. It is also possible to carry out step b) at lower temperatures under vacuum or at a pressure lower than atmospheric pressure. In this case, the temperature may be for example of from 1°C to 20°C, or from 2°C to 15°C, or from 5°C to 10°C, and the pressure may be from 1 to 100 kPa. In any case, evaporating is carried out until the formation of two non-miscible phases or until obtaining a substantially dry solid. This is performed for example when a thin layer salt is deposited on the surface of the biomaterial, or when a solid comprising less than 10% by weight of water is obtained. The formation of two non-miscible phases or a substantially dry solid is visually recognisable. For information, it can be optionally verified by measurement of moisture by gravimetric analysis.

The duration of the evaporation stage may be determined by the skilled person without undue burden, according to his general knowledge. This may be function of the kind of proteins, of salt, of the temperature and the pressure chosen for carrying out the process, of the volume of solution to evaporate, or of the shape of the container. For example, evaporation may be performed from 10 hours to 30 days, for example from 1 day to 20 days, or from 2 days to 30 days. A longer duration may be performed, but it is often without any improvement of the technical features of the biomaterial.

Step b) can be carried out at any pH avoiding the denaturation of proteins, which is known by the skilled person. For example, step b) may be performed a pH comprised between 3.0 and 9.0, the value of 3.0 being optionally excluded. The pH may be for example of between 4.0 and 9.0, or of between 4.0 and 8.0, depending on the kind of proteins.

Evaporation may be carried out by any means meeting the criteria listed above, for example an oven or a vacuum oven.

The process of the invention may consist of steps a) and b) as described above, as they allow obtaining a biomaterial of the invention. In this case, the process does not have any other step, and the biomaterial may be obtained directly at the end of step b), as it may be the substantially dry solid, or the solid phase of the two non-miscible phases obtained in step b).

Alternatively, the process of the invention may comprise additional steps allowing obtaining the biomaterial of the invention. Additional steps may be carried out before step a), and/or between steps a) and b), and/or after step b). In this case, the biomaterial may be obtained after the implementation of these additional steps.

For example, the solid phase or the dry solid obtained in step b) may be washed so that at least a part of the salt is eliminated, thereby obtaining the biomaterial. Preferably, the solid phase or the dry solid obtained in step b) may be washed until elimination of at least 90%wt of the at least one salt, thereby obtaining the biomaterial. The washing may be performed until the elimination of, for example, at least 95%, or at least 99% wt of said at least one salt. The washing may be carried out by any means known by the skilled person, for example with distilled water or aqueous buffer. Control of the resulting salt concentration may be performed with any known method, for example by BCA or microanalysis as illustrated below.

A step of soaking of the solid phase or the dry solid obtained in step b) may be carried out, for example after washing. Soaking may allow hydrating the biomaterial. Soaking may be carried out by any means known by the skilled person, for example with distilled water or a buffer, at room temperature for 48 hours.

It is also possible to add at least one additive during step a) and/or step b), or during any additional step as mentioned above. Additive(s) may be any substance allowing modulating as desired the properties of the biomaterial. Additive(s) may also, in some cases, allow removing the salt or part thereof from the biomaterial. Additive(s) may be chosen by the skilled person according to his general knowledge and to the desired properties for the biomaterial. They may be for example selected among polymers, notably non-charged, positively charged, negatively charged and zwitterionic polymers, non-ionic amino acids and particles. Polymer may be any natural polymer chosen among polysaccharides, proteins, peptides and polynucleotides and/or synthetic and semisynthetic polymers. For example, the polymer includes, but is not limited to, polypeptides, homopolypeptides, chitosan, hyaluronic acid, heparin, alginate, chondroitin sulfate, polyarginine, polylysine, ε-polylysine, DEAE dextran, polycyclodextrine, polyallylamine hydrochloride, polyethylenimine, xanthan gum, polyacrylic acid, polyethylene glycol, starch, cellulose and its derivatives, collagen, insulin, fibrinogen, casein, gelatin, gliadin, gluten, elastin, globulin and haemoglobin. The amino acid may be chosen among all suitable amino acids, and preferably among arginine, ornithine, lysine and cysteine. The particle may be any suitable particle, and may be for example selected among nanoparticles, for example carbon nanotubes or graphene, microparticles, bacteria and viral vectors. The additive may be incorporated by any means known by the skilled person. For example, it can be incorporated in the solution obtained in step a) by dissolving and/or suspending said additive directly in the solution obtained in step a), or by dissolving and/or suspending said additive in a water-miscible solvent, then adding the mixture to the solution obtained in step a). Additionally, or alternatively, the additive may be incorporated in the biomaterial obtained in step b) by adsorption of said additive dissolved and/or suspended in a solvent onto the biomaterial obtained in step b). The quantity of additive may be adapted to the proteins and to the desired properties of the biomaterial, and therefore may be determined by the skilled person according to his general knowledge. For example, the percentage of additive may be from 0 to 20% wt, with respect to the total quantity from proteins in the biomaterial, for example from 1 to 18% wt, or from 2 to 15% wt, or from 3 to 12% wt.

In addition or alternatively, at least one active ingredient may be incorporated in the solution obtained in step a) and/or in the biomaterial obtained in step b). This may allow functionalizing the biomaterial. The active ingredient may be incorporated by any means known by the skilled person. For example, it can be incorporated in the solution obtained in step a) by dissolving and/or suspending said active ingredient directly in the solution obtained in step a), or by dissolving and/or suspending said active ingredient in a water-miscible solvent, then adding the mixture to the solution obtained in step a). Additionally or alternatively, the active ingredient may be incorporated in the biomaterial obtained in step b) by adsorption of said active ingredient dissolved and/or suspended in a solvent onto the biomaterial obtained in step b). In this case, the solvent may be selected among water, organic solvents or a mixture of water and a water-miscible solvent. Active ingredient(s) may be any substance allowing confering interesting properties to the biomaterial. Active ingredient(s) may be chosen by the skilled person according to his general knowledge and to the desired properties for the biomaterial. It may be selected among anti-cancer substances, such as goserelin, leuprolide, carmustine, paclitaxel, histrelin or gemcitabine, anti-inflammatory agents, such as diclofenac, immunosuppressants such as azathioprine or methotrexate, immunomodulators such as cyclosporine, modulators of cell-extracellular matrix interaction including cell growth inhibitors such as imatinib or axitinib, anticoagulants such as rivaroxaban or edoxaban, antithrombotic agents such as clopidogrel, enzyme inhibitors, analgesic such as morphine or hydrocodone, antiproliferative agents, antimycotic substances, cytostatic substances, growth factors such as erythropoietin or thrombopoietin, enzymes, hormones, steroids such as hydrocortisone or prednisolone, non-steroidal substances, and anti-histamines such as diphenhydramine or fexofenadine, this list not being limitative. The quantity of active ingredient may be adapted to the proteins and to the desired properties of the biomaterial, and therefore may be determined by the skilled person according to his general knowledge. For example, the percentage of active ingredient may be of from 0 to 25% wt, with respect to the total quantity of proteins in the biomaterial, for example from 1 to 20% wt, from 1 to 18% wt, or from 2 to 15% wt, or from 3 to 12% wt.

In any case, the biomaterial obtained by the process of the invention may contain at least 50% wt of proteins, with respect to the total weight of the biomaterial, for example at least 55%, or at least 60%, or at least 70%, or at least 80%, or at least 90%, or even 100%.

The biomaterial obtained by the implementation of the preparation process is a second object of the invention.

As explained above, the properties of the biomaterial, including its visual appearance, may be modulated by modifying one or more of the parameters of the process of preparation, notably the kind of salt and the ratio salt/protein, thereby offering the possibility to modulate as desired the properties of the biomaterial.

Regarding the firmness of the biomaterial, it may be a solid, insoluble biomaterial, ranging from foam to compact material, including hydrogels.

Regarding the shape of the biomaterial, it may be in any desired shape, depending on the envisaged application and on the container used to prepare the biomaterial. It may be a membrane, a tube, a cylinder, a pad, a ring, this list not being limitative. The biomaterial may also be cut after implementation of the process in order to obtain the desired shape or size.

The biomaterial may be in any desired size, including microparticles, by grinding the biomaterial.

The visual aspect of the biomaterial may be translucent to opaque.

As mentioned above, one of the major advantages of the biomaterial of the invention is that the proteins used to prepare the biomaterial are not denatured during the process of preparation of the invention. The preparation is performed in non-denaturing conditions and the shape and/or the secondary structures of the proteins may be analysed in the solution obtained in step a) and in the biomaterial obtained in step b). "Not denatured" means, according to the invention, that the percentage of secondary structures of said protein in the solution obtained in step a) is at least the same as in a control solution prepared with the corresponding native protein in a similar concentration, and that the percentage of secondary structures of said protein in the biomaterial obtained in step b) presents at least a substantial increase of β-turns and intermolecular β-sheets and a substantial decrease of unordered structures in comparison to the corresponding native protein. The shape and secondary structures of the protein in the biomaterial may be controlled by any method known by the skilled person, for example by IR analysis or SAXS, as illustrated thereafter.

The biomaterial of the invention is particularly stable over time. Advantageously, it is stable in aqueous solutions, in acidic, neutral and basic pH, and/or in organic solvents such as ethanol, during at least 2 days, and preferably at least 7 days. This means that there is substantially no dissolution of the biomaterial during this period, even if alteration of its structure by breakage of hydrogen bonding and disulfide bridges, and a mass loss lower than 20%, can be observed. For example, the mass loss may be up to 20% in basic solution, and up to 10% in water, ethanol and acidic solutions. Mass loss of the biomaterial may be calculated as illustrated in Example below.

As explained above, the biomaterial may be made only of proteins, notably non-denatured proteins, which imply a great biocompatibility. Advantageously, the biomaterial may be associated with at least one active ingredient as illustrated before in order to improve its biological properties.

Therefore, another object of the invention relates to the use of the biomaterial of the invention as a support for tissue engineering *in vitro* and/or for *in vitro* cell culture and expansion and/or an implantable medical device. Indeed, the biomaterials of the invention provide necessary structural and biochemical support for cell growth and as they may be three dimensional, they are particularly suitable for cell culture and drug/cell delivery.

Another object of the invention relates to the use of the biomaterial for use as a drug. As the biomaterial of the invention is able to interact with biological systems, it may for example be used for the constitution of a device for diagnostic purposes, of a tissue or organ substitute or of a device of functional substitution. Therefore, the biomaterial of the invention may be used *in vivo* as an implantable medical device, notably to replace defective tissues in a subject in need thereof, or for a drug release system. In other words, the invention also describes an implantable medical device comprising the biomaterial of the invention. Advantageously, the device or drug comprising the biomaterial of the invention may be resorbing after a period into a living body, depending on the nature of the biomaterial. For example, the period may be after 20 days after insertion into a living body, or 30 days, or more than 60 days.

This invention is further illustrated by the following examples with regard to the annexed drawings that should not be construed as limiting.

**Brief description of the figures**

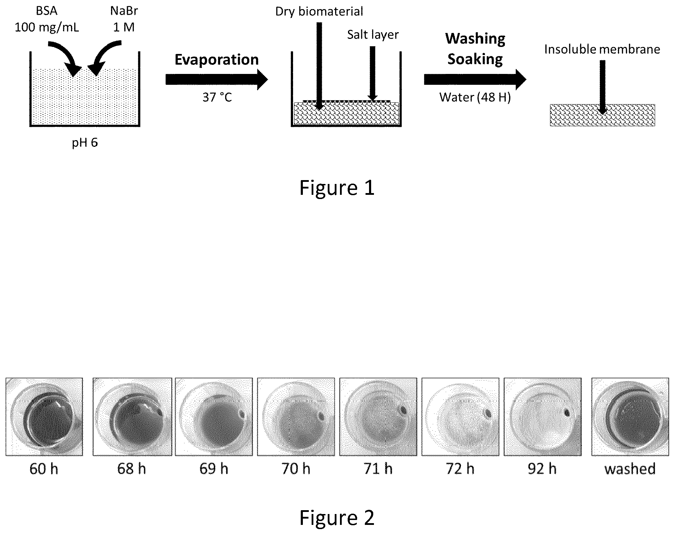

- Figure 1 represents the preparation of albumin-based biomaterials by evaporation in the presence of salt. The molar ratio NaBr/BSA (bovine serum albumin) of the selected formulation is 664. The evaporation is carried on in an oven (37 °C) under atmospheric pressure during 7 days until the biomaterial is completely dry. The excess salt forms a thin layer on the surface of the biomaterial. After evaporation, washing and soaking steps are applied to remove the salt, leaving a water insoluble albumin-based biomaterial.

• Figure 2 represents the tracking of the formation of a BSA/NaBr 664 membrane (evaporation at 37 °C, pH = 6, M/S = 105 mg/cm$^2$) over time. Once washed, the membrane is transparent. The bottom of the mold was covered with a non-stick silicone disk. The irregularity present in the upper right corner is due to an air bubble.

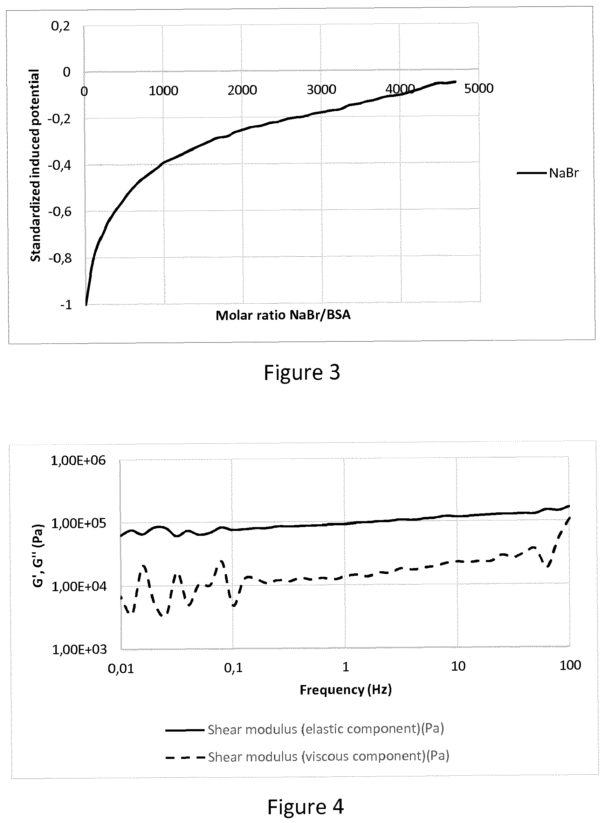

• Figure 3 represents the surface charge titration of albumin in the presence of an increased concentration of NaBr (pH = 6).

• Figure 4 represents the rheological properties of albumin-based membranes by an oscillation protocol (frequency ramp: 100 Hz to 0.01 Hz, shear stress controlled: 1 Pa). Elastic component of shear modulus (Pa) is represented by a solid line; viscous component of shear modulus (Pa) is represented by a dashed line.

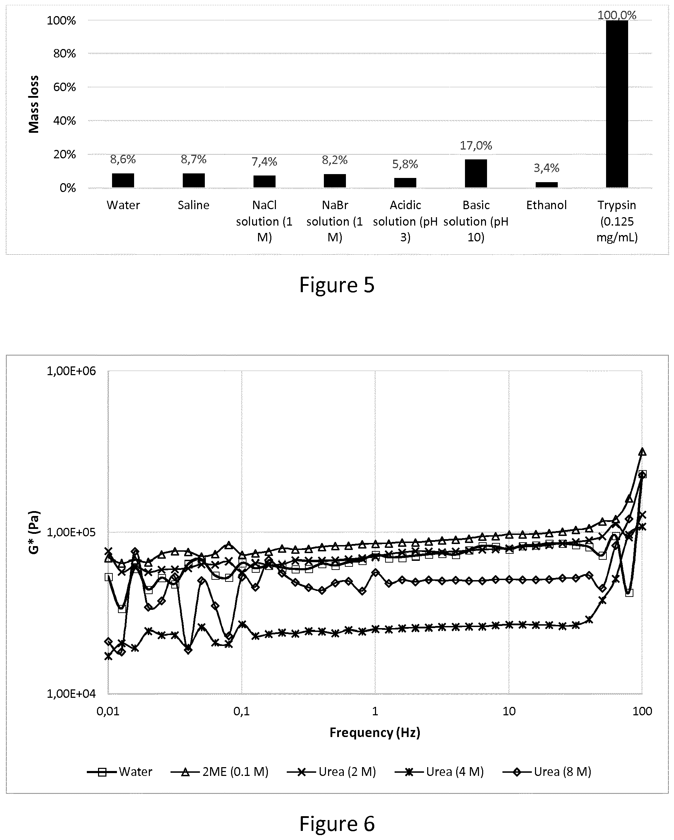

• Figure 5 represents the stability of BSA/NaBr 664 membranes (evaporation at 37 °C and pH = 6) in various dissolution media (from the left to the right: water, saline, NaCl solution 1M, NaBr solution 1M, acidic solution pH 3, basic solution pH 10, ethanol and trypsin 0.125 mg/ml). For each dissolution media, membranes were placed in 25 mL of media. The experiments were performed at 37 °C and under stirring during 7 days.

• Figure 6 represents the rheological properties of BSA/NaBr 664 membranes (evaporation at 37 °C and pH = 6) incubated in distilled water ( ▭ ), a solution of 2-mercaptoethanol (2ME, 0.1 M, ▲ ) and solutions of urea (2 M ✕ , 4 M ✳ and 8 M ◆ ). For solution, a batch of 2 membranes is placed in 30 mL of media.

The experiments were performed at room temperature for 24 h. The rheological properties of the membranes were assessed by an oscillation protocol (frequency ramp: 100 Hz to 0.01 Hz, shear stress controlled: 1 Pa).

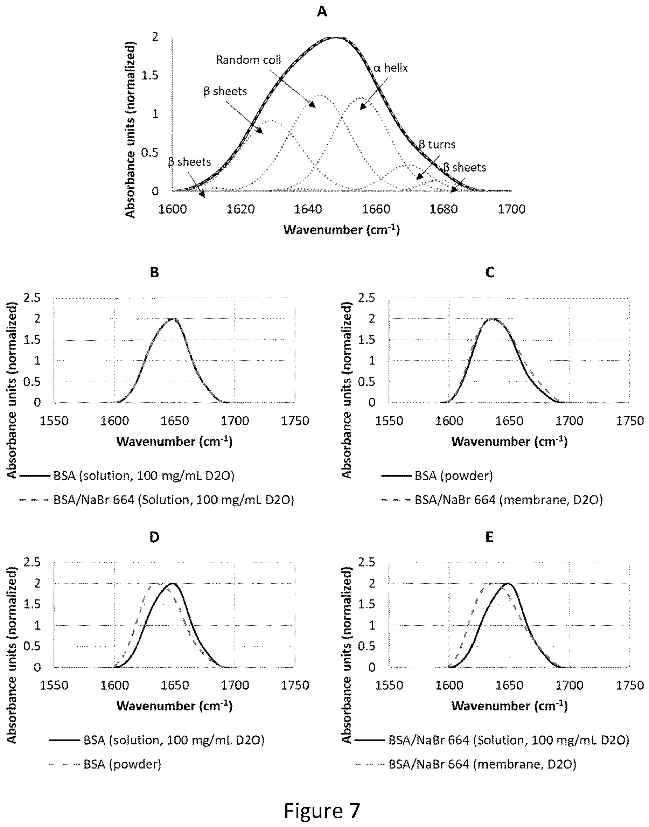

• Figure 7 represents (A) the fitting of the amid I band of albumin present in a BSA solution (100 mg/mL, in $D_2O$) and identification of the subbands of each secondary structure (the fitting curve and the original amid I band spectra are overlapping, residual RMS error < 0.005) and (BE) a comparison of the amid I bands of BSA in (B) a BSA control solution (100 mg/mL, $D_2O$, solid line) and a BSA/NaBr 664 solution (BSA: 100 mg/mL, NaBr: 1M, $D_2O$, dashed line), (C) a BSA control powder (solid line) and a BSA/NaBr 664 membrane (dashed line), (D) a BSA control solution (100 mg/mL, $D_2O$, solid line) and a BSA control powder (dashed line) and (E) a BSA/NaBr 664 solution (BSA: 100 mg/mL, NaBr: 1M, $D_2O$, solid line) and a BSA/NaBr 664 membrane (dashed line).

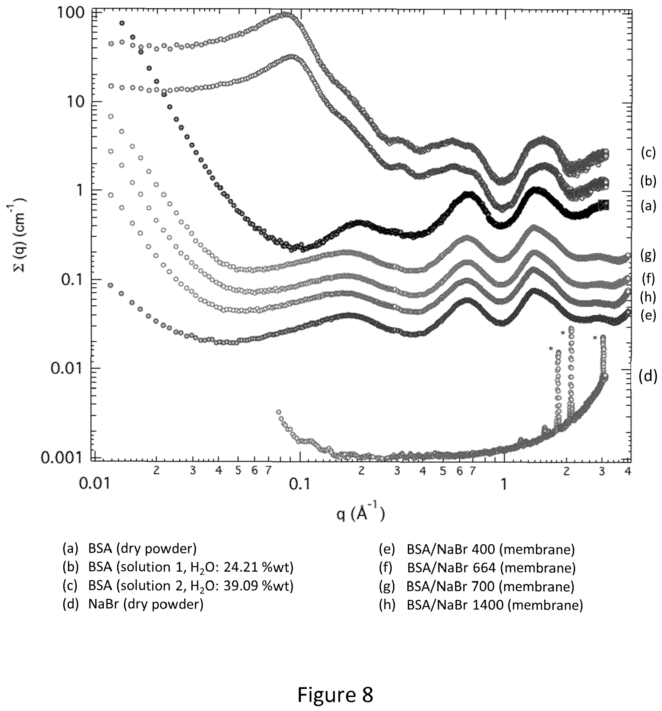

• Figure 8 represents the scattering curve obtained by SAXS analysis of (a) dry powder of BSA, (b, c) solutions of BSA ((b) $H_2O$: 24.21 %wt, (c) $H_2O$: 39.09 %wt), (d) dry powder of NaBr, (e) BSA/NaBr 400 membrane, (f) BSA/NaBr 664 membrane, (g) BSA/NaBr 700 membrane, (h) BSA/NaBr 1400 membrane.

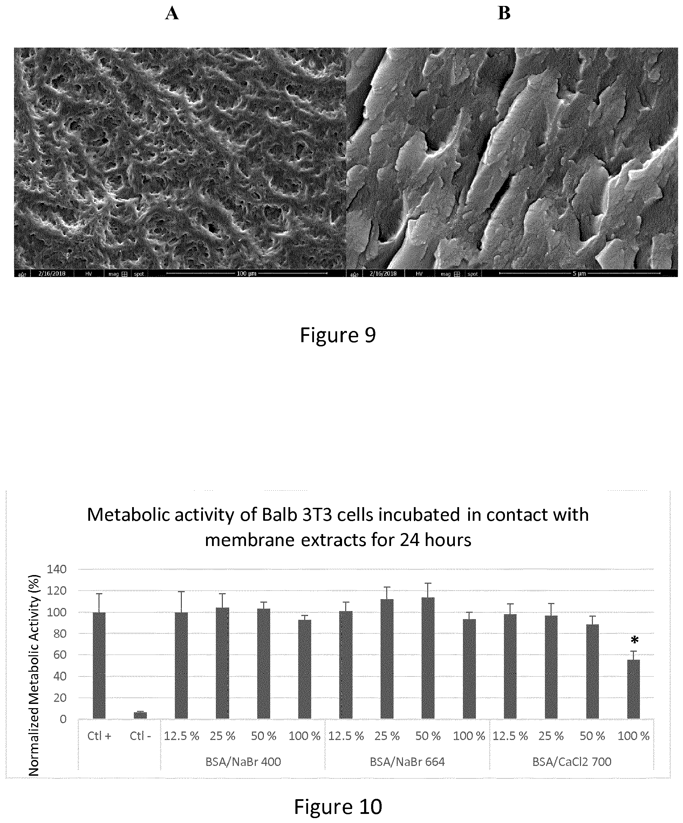

• Figure 9 represents SEM analysis of an albumin-based membrane formulated with a molar ratio NaBr/BSA of 664 under the selected conditions (evaporation at 37 °C and pH = 6). The samples were metallized before observation. A) Surface of membrane; B) cross-section of the membrane.

• Figure 10 represents cell viability of Balbc 3T3 fibroblasts treated with membrane extracts (BSA/NaBr 400, BSA/NaBr 664 and BSA/CaCl$_2$ 700, at 12.5%, 25%, 50% and 100%). The indirect cytotoxicity is estimated by comparing the normalized metabolic activity of Balbc 3T3 cells cultivated during 24 hours in contact with BSA/NaBr 400, BSA/NaBr 664 and BSA/CaCl$_2$ 700 extracts with the normalized metabolic activity of the positive control (Ctl+). (*) A significant difference was observed between the treated groups and the positive control (Ctl+) (p < 0.05). Biological replicates = 1, total technical replicates = 4.

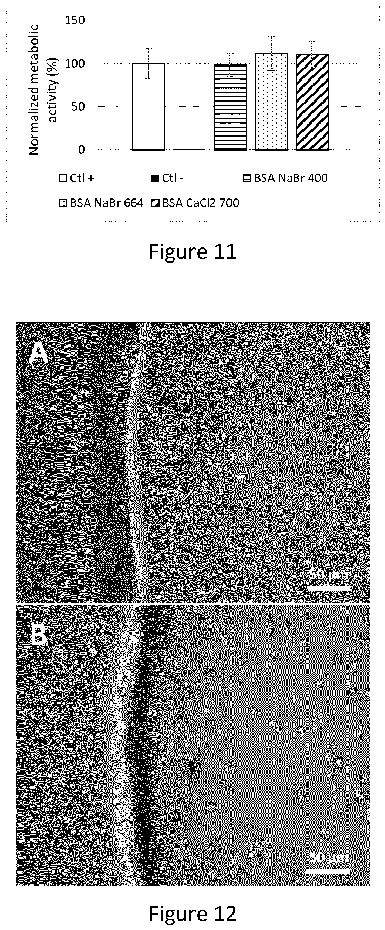

• Figure 11 represents cell viability of Balbc 3T3 fibroblasts cultivated in direct contact with albumin membranes. The direct cytotoxicity is estimated by comparing the normalized metabolic activity of Balbc 3T3 cells cultivated during 24 hours in contact with BSA/NaBr 400 ( ⊟ ), BSA/NaBr 664 ( ⊡ ) and BSA/CaCl$_2$ membranes ( ▨ ) (M/S = 25 mg/cm$^2$) with the normalized metabolic activity of the positive control (Ctl+). No significant difference was observed between the Ctl+ (□) group and the treated groups. Biological replicates = 4, total technical replicates = 20.

• Figure 12 represents microscopic examination of Balbc 3T3 mouse fibroblast cultivated during 24 hours in contact with BSA/NaBr membranes. Fibroblasts can be seen around (A) and above (B) the biomaterial.

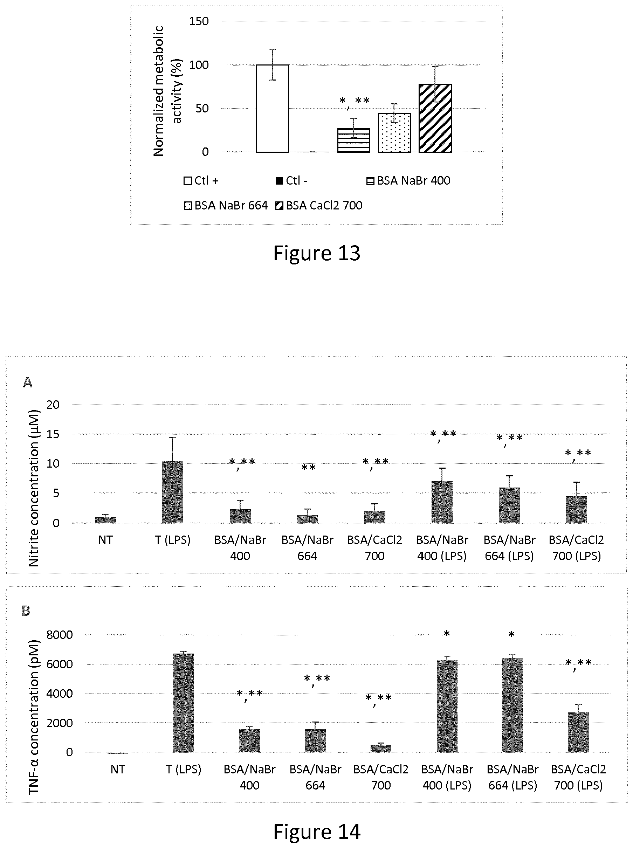

• Figure 13 represents normalized metabolic activity of Balbc 3T3 fibroblasts measured on albumin membranes (BSA/NaBr 400, BSA/NaBr 664, and BSA/NaCl$_2$ 700, respectively ⊟ , ⊡ , ▨ ) freshly transferred to empty unused wells after elimination of the culture media. The cell adhesion is estimated by comparing the normalized metabolic activity of Balbc 3T3 cells between the treated and the untreated groups. (*) A significant difference was observed between the treated groups group and the positive control (Ctl+, □) (p < 0.05). (**) A significant difference was observed between the treated groups (p < 0.05). Biological replicates = 3, total technical replicates = 12.

• Figure 14 represents RAW macrophages cultivated during 48 h at 37 °C in contact with BSA membranes (M/S = 25 mg/cm$^2$, from the left to the right: BSA/NaBr 400, BSA/NaBr 664, BSA/CaCl$_2$ 700, BSA/NaBr 400 LPS, BSA/NaBr 664 LPS, BSA/CaCl$_2$ 700 LPS). Nitrite (A) and TNF-$\alpha$ (B) concentrations were measured to assess the activation of macrophages and the inflammatory response. The non-treated group (NT) was cultivated without membranes and without LPS. The LPS was added after 24 h in the culture media (50 ng/mL) in the LPS-treated control group (T LPS) and the LPS-activated groups (LPS). (*) A significant difference was observed between the treated groups and the NT group (p < 0.05). (**) A significant difference was observed between the treated groups and the T(LPS) group (p < 0.05). Biological replicates = 3, total technical replicates = 12.

## Examples

### Materials

### Chemical reagents.

Bovine serum albumin (fraction V, ≥96%) was purchased from Acros Organics. Human serum albumin (≥ 96%), Ovalbumin (≥ 98%) and Gamma-globulins from bovine blood (≥99%) were purchased from Sigma-Aldrich.

Sodium bromide (NaBr), Potassium chloride (KCl) and Potassium acetate (KC$_2$H$_3$O$_2$) were purchased from Sigma-Aldrich. Sodium chloride (NaCl) was purchased from VWR Chemicals. Potassium bromide (KBr) was purchased from Acros Organics. Sodium iodide (NaI) and Dipotassium phosphate (K$_2$HPO$_4$) were obtained from Prolabo. Potassium iodide (KI) was purchased from Carbo Erba Reagents. Magnesium chloride (MgCl$_2$, anhydrous) and Ammonium formate (NH$_4$HCO$_2$) were purchased from Fluka. Calcium chloride (CaCl$_2$, 2H$_2$O) was purchased from Merck. Potassium car-

bonate ($K_2CO_3$) was purchased from Alfa Aesar.

BCA Assay reagents (Bicinchoninic acid solution and Copper(II) sulfate pentahydrate) were purchased from Sigma-Aldrich. Deuterium oxide ($D_2O$) was purchased from Sigma-Aldrich.

**Biological reagents**

Balbc 3T3 mouse fibroblasts (clone A31 ATCC® CCL-163) were cultivated in Dulbecco's Modified Eagle Medium High Glucose (DMEM) containing stabilized glutamine and sodium pyruvate (Dutscher), supplemented with 10% (v/v) of fetal bovine serum (Dutscher) and 1% (v/v) of Penicillin-Streptomycin Solution 100X (final concentrations : 0.06 mg/mL and 0.1 mg/mL respectively) (Dutscher) at 37 °C in 5% $CO_2$, 95% humidity. Cells were harvested using trypsin (0.5 g/L)-EDTA (0.2 g/L) (Dutscher) for 5 min at 37 °C. Thiazolyl Blue Tetrazolium Bromide (MTT) was purchased from Sigma-Aldrich. CellTiter Glo® Viability Assay was purchased from Promega.

RAW 264.7 mouse macrophages (ATCC® TIB-71™) were cultivated in Dulbecco's Modified Eagle Medium High Glucose (DMEM) containing stabilized glutamine (Sigma-Aldrich), supplemented with 5% (v/v) of heat-inactivated fetal bovine serum (Gibco), Penicillin (100 U/mL) (Sigma-Aldrich) and Streptomycin (0.1 mg/mL) (Sigma-Aldrich) at 37 °C in 5% $CO_2$, 95% humidity. Cells were harvested using trypsin (0.5 g/L)-EDTA (0.2 g/L) (Sigma-Aldrich) for 5 min at 37 °C. Lipopolysaccharide (LPS) from *Escherichia coli* (K12) was purchased from Invivogen. Purified anti-mouse TNF-$\alpha$ antibody clone 1F3F3D4 and biotinylated anti-mouse TNF-$\alpha$ antibody clone XT3/XT22 for ELISA testing were purchased from eBioscience/ThermoFisher Scientific. Horseradish Peroxidase Avidin (Avidin HRP) was purchased from Jackson. P-aminobenzenesulfonamide and acetic acid purchased from sigma. N-(1-naphtyl)ethylenediamine dihydrochloride was purchased from Acros Organics.

**Methods**

**Formulation (general procedure)**

A solution of BSA (100 mg/mL) and NaBr 1 M (molar ratio NaBr/BSA = 664) in an acetate buffer (0.2 M) at pH 6 was prepared. This solution was deposited in a mold (bottom covered with a non-stick silicone disk) and evaporated at 37 °C for 7 days. The dry biomaterial obtained was washed to remove the salt and soaked in distilled water at room temperature for 48 hours. The water-insoluble membrane (BSA/NaBr 664) was then collected and characterized.

**Initial characterization**

The molar ratio salt/albumin (equation 1) and the ratio M/S (equation 2) were used to label the formulations. The relative yield (equation 3), the water absorption (equation 4) and the initial expansion (equation 5) were used to compare the formulated membranes. The density (equation 6) of the material was assessed by immersion of the material in distilled water at room temperature. WBSA represents the initial weight of albumin used for the formulation. Ai represents the area of the container used during the evaporation process. Wd represents the weight of the final dried membrane after washing in distilled water for 48 h and drying in an oven at 37 °C overnight. Vd is the volume of the dried membrane measured by immersion of the material in distilled water at room temperature. Wh represents the weight of the hydrated membrane at equilibrium after immersion in distilled water for 24 h and removal of excess surface water using filter paper. Ah is the area of the hydrated membrane's surface calculated after measuring its diameter using an electronic digital caliper (TACKLIFE-DC01, accuracy $\pm$ 0.2 mm).

$$Molar\ ratio\ salt/albumin = \frac{Molar\ concentration\ (salt)}{Molar\ concentration\ (albumin)} \quad (1)$$

$$M/S = \frac{W_{BSA}}{A_i} \quad (2)$$

$$Relative\ yeild\ (Y\%) = \frac{W_d}{W_{BSA}} \times 100 \quad (3)$$

$$Water\ absorption\ (W\%) = \frac{W_h - W_d}{W_d} \times 100 \qquad (4)$$

$$Initial\ expansion\ (E\%) = \frac{A_h}{A_i} \times 100 \qquad (5)$$

$$Density = \frac{W_d}{V_d} \qquad (6)$$

**Standardized induced potential (SIP) measurements**

The titrated solution was prepared by dissolving BSA in mQ water at a concentration of 1 mg/mL. The titration solution was prepared by dissolving NaBr in mQ water at a concentration of 60 mg/mL. The NaBr solution was then used to titrate the protein's surface charge by measuring the induced potential using streaming current detection. A Mütek PCD 02 detector was used. 10 mL of the BSA solution was transferred to the detector tank. Then, after an equilibration time of 5 minutes, consecutive additions of the saline NaBr solution with a frequency of 30 $\mu$L/min were performed. The assay was stopped when the measured potential reached a plateau.

**BCA assay**

BSA/NaBr 664 membranes (initial BSA concentration = 200 mg/mL, M/S = 105 mg/cm$^2$, n = 3) were washed with 4.5 mL of ultrapure water each (2x1.5 mL (2x30 min) then 1x1.5 mL (2 h)). Then, for each rinsing solution, the volume was adjusted to 5 mL using a volumetric flask. Albumin concentration was then determined in the initial solution using in the formulation as well as in the rinsing solution by a BCA test using a standard range (20 $\mu$g/mL - 1000 $\mu$g/mL). The assay was carried out in a 96-well plate. The reagent (bicinchoninic acid / CuSO$_4$) was added to the solutions (200 $\mu$L of reagent to 25 $\mu$L of protein solution). Then, the plate was incubated at 37 °C for 30 minutes. The absorbance reading at 560 nm was performed at room temperature using a SAFAS Xenius XM spectrofluorometer (SAFAS Monaco). After calculating the quantity of albumin in the membrane, the quantity of NaBr was deduced.

**Microanalysis**

Electron-excited X-ray microanalysis was performed on randomly selected areas of the samples using a Quanta™ 250 ESEM (FEI Company, Eindhoven, The Netherlands) operating with an accelerated voltage of electrons of 10 kV (emergence angle = 35 °, acquisition time = 100 s, process time = 7.68 $\mu$s). Four BSA/NaBr membranes were analysed: BSA/NaBr 400, BSA/NaBr 664, BSA/NaBr 700 and BSA/NaBr 1400 (M/S = 113 mg/cm$^2$). The amount of NaBr was estimated using the atomic percentage of brome in the samples.

**Rheological behaviour and compression assay**

The rheological characterization and the compression assay were performed using a Malvern Kinexus ultra + rheometer equipped with a plane mobile of 2 cm in diameter. Hydrated BSA/NaBr 664 membranes (M/S = 105 mg/cm$^2$, thickness = 1.7 mm) were used. For the oscillation protocol, the samples were subjected to a controlled shear stress of 1 Pa and the measurements were carried out according to an oscillation frequency ramp ranging from 100 Hz to 0.01 Hz at 25 ° C. For the compression assay, a force ramp of 0.5 N to 40 N (0.04 mm/s) was applied to the samples at 25 °C. The elastic modulus (E) of each sample was calculated within the elastic domain of the strain ($\varepsilon$)-stress ($\sigma$) curve (equation 7).

$$\sigma = E \times \varepsilon \qquad (7)$$

**Traction assay**

The traction assay was performed using an Instron ElectroPulsTM E3000 equipped with a 100 N force sensor.

A batch of six hydrated BSA/NaBr 664 membranes (M/S ratio of 110.9 mg/cm$^2$) was used. The samples were cut with a punch to form six specimens with standardized dimensions (initial useful length Lo = 40 mm, effective initial width $l_0$ = 10 mm, initial thickness eo = 1.74 $\pm$ 0.079 mm). The tensile test was then carried out at room temperature with a tensile speed of 0.1 mm/s. The elastic modulus (E) was calculated within the elastic domain of the strain ($\varepsilon$)-stress ($\sigma$) curve (equation 7).

**Stability in aqueous solutions**

BSA/NaBr 664 membranes (M/S = 105 mg/cm$^2$) were placed, in batches of 3, in 25 mL of the following dissolution media: distilled water, physiological saline (0.9 % NaCl), acidic medium (pH 3, colour indicator: bromothymol blue), basic medium (pH 10, colour indicator: bromothymol blue), saline solution of 1 M NaCl, saline solution of 1 M NaBr, ethanol and a trypsin solution (0.125 mg/mL, diluted in a PBS buffer). The media containing the membranes was then incubated at 37 °C with shaking (180 rpm) for 7 days. After that, the membranes were washed with water before being characterized for their mass loss (equation 8).

$$Mass\ loss\ (ML\%) = \frac{Initial\ weight - Final\ weight}{Initial\ weight} \times 100 \quad (8)$$

**IR analysis**

A VERTEX 70 FTIR spectrometer (Bruker, Germany) equipped with a deuterated tryglycine sulphate detector (RT DLaTGS) and a KBr beamsplitter, was employed for infrared measurements. D$_2$O solutions were used to avoid the spectral overlaps between Amide I band and strong absorption band of water at approximately 1650 cm$^{-1}$. All samples were placed between two CaF$_2$ windows. The FTIR spectra of the samples have been recorded at room temperature between 4000 and 800 cm$^{-1}$ at 2 cm$^{-1}$ nominal resolution, accumulating 128 scans per spectrum and with a scanning rate of 10 kHz, taking D$_2$O spectrum as background. The liquid samples (solutions of BSA, BSA/NaBr 400 and BSA/NaBr 664) were prepared in D$_2$O with a BSA concentration of 100 mg/mL. The solid samples (membranes of BSA/NaBr 400 and BSA/NaBr 664) were hydrated with D$_2$O.

Spectral analysis was performed by using the spectrometer software OPUS 7.5 (Bruker, Germany). For secondary structure analysis, a curve fitting method was performed for the amide I region (1700-1600 cm$^{-1}$) of the deconvolved spectra. Prior to curve fitting, the spectra were baseline-corrected for the amide I band using the minima at the low wavenumber (1600 cm$^{-1}$) and high wavenumber (1700 cm$^{-1}$) sides. Deconvolution was carried out according to the least-square iterative curve fitting program (Levenberg-Marquardt) using a Gaussian line-shape. The number of subbands and their positions were determined from the deconvolved spectra as well as from the second and fourth derivative of the spectra. For the final fits, in order to reduce the residual RMS error as much as possible (less than 0.005), heights, widths and positions of all bands were adjusted while at least one of these parameters was not allowed to change each time. Finally, second derivatives of the original and the fitted curve were compared to ensure the accuracy of curve fitting. The fractional areas of the fitted components were used to calculate the percentages of different secondary structure elements ($\alpha$ helix, $\beta$ sheets, $\beta$ turns, and random coils).

**SAXS ICS**

SAXS analysis were performed on dry membranes (BSA/NaBr 400, BSA/NaBr 664, BSA/NaBr 700 and BSA/NaBr 1400) as well as on three controls: a dry powder of lyophilized BSA and two solutions of BSA (solution 1: 113.69 mg BSA + 36.31 mg H$_2$O, solution 2: 119.39 mg BSA + 79.61 mg H$_2$O). The data was integrated into a 1D scattering intensity $\Sigma(q)$, as a function of the magnitude of the scattering vector q (equation 9) where $\theta$ is the total scattering angle.

$$q = \frac{4\pi}{\lambda} \sin\left(\frac{\theta}{2}\right) \quad (9)$$

**Scanning electron microscopy (SEM)**

Scanning electron microscopy assessments were performed using a Quanta™ 250 ESEM (FEI Company, Eidhoven, The Netherlands) operating with an accelerated voltage of electrons of 10 kV. BSA/NaBr 664 membranes (M/S ratio = 105 mg/cm$^2$) were dried at 37 °C during 48 hours. The samples were then coated with a gold-palladium alloy using a Hummer Jr sputtering device (Technics, Union City, CA, USA). The surface and the cross section were examined.

**Indirect cytotoxicity test**

The membranes used during this test were BSA/NaBr 400, BSA/NaBr 664 and BSA/CaCl$_2$ 700. The membranes were formulated with a ratio M/S of 25 mg/cm$^2$. The membranes were washed with ethanol 70 % then with sterile PBS 1X and sterilized for 15 minutes under UV light. Then, they were stored in sterile PBS 1X until further use. For the indirect cytotoxicity assessment, each membrane was transferred to a 12-well plate and extracted in 1.5 mL of culture medium (DMEM + FBS (10 %) + PS (1%)) under stirring at 37 °C during 72 h. Dilutions containing 12.5%, 25%, 50% and 100% (v/v) of the extracts were then prepared. Balbc 3T3 mouse fibroblasts (clone A31 ATCC® CCL-163) were cultivated in a 96-well plate at 8000 cells per well (culture medium: DMEM + FBS (10 %) + PS (1%)) at 37° C for 24 h. The following day, the culture medium in each well was replaced with 100 μL of the diluted extracts. A positive and a negative control were prepared with only the culture media and with the culture media containing 20 % of DMSO respectively. The plate was then incubated at 37°C for 24 h. After incubation, the culture medium in each well was replaced with 100 μL of a solution of MTT diluted in fresh culture medium (1 mg/mL) and the plate was incubated at 37°C for 2 h. The formazan crystals were then solubilized in 80 μL of DMSO and the absorbance at 560 nm was measured using the SAFAS apparatus after an equilibration of 15 min at room temperature. The metabolic activity of the positive control was used to determine the percentage of viable cells in each group.

**Direct cytotoxicity test**

The membranes used during this test were BSA/NaBr 400, BSA/NaBr 664 and BSA/CaCl$_2$ 700. The membranes were formulated with a ratio M/S of 25 mg/cm$^2$ in non-stick silicone molds. The hydrated membranes were cut using a circular punch to obtain small disks (diameter = 5 mm, thickness = 0.7 mm). The disks were washed with ethanol 70 % then with sterile PBS 1X and sterilized for 15 minutes under UV light. Then, they were stored in sterile PBS 1X until further use. For the direct cytotoxicity assessment, the sterilized disks were transferred to a black-walled 96-well plate. Balbc 3T3 mouse fibroblasts (clone A31 ATCC® CCL-163) were then added to the plate at 8000 cells per well (culture medium: DMEM + FBS (10 %) + PS (1%)) directly on the disks of biomaterials. A positive and a negative control were added with only the culture media and with the culture media containing 20 % of DMSO respectively. The plate was then incubated at 37° C for 24 h. After incubation, the plate was equilibrated at room temperature for 30 minutes. The culture media was eliminated. In each well, 50 μL of new culture media was added followed by 50 μL of the CellTiter-Glo® reagent. The bioluminescence was then measured using the SAFAS apparatus and the following protocol: the plate was stirred for 2 minutes then left to equilibrate for 10 minutes before measuring the bioluminescence. The metabolic activity of the positive control was used to determine the percentage of viable cells in each group.

**Macrophage activation assay**

The membranes used during this test were BSA/NaBr 400, BSA/NaBr 664 and BSA/CaCl$_2$ 700. The membranes were formulated with a ratio M/S of 25 mg/cm$^2$ in non-stick silicone molds. The hydrated membranes were cut using a circular punch to obtain small disks (diameter = 5 mm, thickness = 0.7 mm). The disks were washed with ethanol 70 % then with sterile PBS 1X and sterilized for 15 minutes under UV light. Then, they were stored in sterile PBS 1X until further use. For the macrophage activation assay, the sterilized disks were transferred to a 96-well plate. RAW 264.7 macrophages were then added to the plate at 100000 cells per well (culture medium: DMEM + FBS (5 %) + PS (1%)) directly on the disks of biomaterials. After 24 h of incubation at 37 °C, LPS was added to the LPS-treated groups to obtain a final concentration of 50 ng/mL in each well. The plate is then incubated for another 24 h at 37 °C. A negative and a positive control were prepared with only the culture media and with the culture media containing 50 ng/mL of LPS respectively. The shapes of the cells were then evaluated by microscopy and NO and TNF-α production were assessed as follows.

**Assessment of NO production**

Concentrations of nitrite in cell supernatants were evaluated by Griess test (n=3). 60 μL of Griess reagent (v/v mixture of 58.1 mM p-aminobenzene sulfonamide in 30 % acetic acid and 3.9 mM N-(1-naphtyl)ethylenediamine dihydrochloride in 60 % acetic acid) were added to 40 μL of supernatant and the absorbance at 543 nm was measured and compared to a sodium nitrite standard curve.

**Assessment of TNF-α production**

TNF-α concentration in cell supernatants was evaluated by ELISA (n=3) using commercially available reagents and following the manufacturer instructions. Capture antibody was diluted to 1 μg/mL in a 0.05 M pH 9.6 carbonate/bicarbonate buffer and coated 1 night at 4 °C before blocking with PBS 0.05 % Tween 20 1 % BSA (1 h, 37 °C). Samples were then diluted with culture media and incubated with capture antibody (2 h, 37 °C) before detection antibody diluted to 0.5 μg/mL in PBS 0.05 % Tween 20 1 % BSA was added (1 h, 37 °C). Avidin HRP was then introduced (45 min, 37 °C) and revelation was conducted by adding a solution of 1.25 mM tetramethylbenzidine and 13.05 mM $H_2O_2$ in 0.1 M pH 5 citrate buffer. Revelation was finally stopped by addition of 1 M HCl and absorbance was measured at 450 nm.

**Statistical analysis**

Data were analysed by using R (version 3.6.1, R Foundation for Statistical Computing, Vienna, Austria). The normality of distribution was determined with the Shapiro-Wilk test. The equality of variances was determined with the F-test. When the data were normally distributed and the variances of the samples were equal, a t-test (2-tailed) was used to compare two means. When these two conditions did not apply, a Mann-Whitney test was performed instead. Values were considered statistically significant at $p < 0.05$.

**Results and discussion**

**Formulation parameters screening**

The solubility of albumin in aqueous solutions and its thermal and pH-stability regions were thoroughly studied. Previous studies showed that albumin is stable in a range of pH between 3 and 9. It was also shown that the denaturation temperature is dependent on the pH and decreases at low pH values (62 °C at pH 7.4, 46.8 °C at pH 3.5). In this work, the evaporation temperature was set to 37 °C and the pH to 6 in order to preserve as much as possible the native structure of albumin. Then, a thorough screening of operational conditions was carried out to identify those allowing the formation of interesting biomaterials. Albumin solutions (protein alone or with salt) formulated at controlled pH (pH = 6) were evaporated in an oven at 37 °C until the residues were completely dry (see figure 1). Afterwards, the residues were washed to eliminate the excess salt and protein, then soaked in distilled water for 48 hours to assess their water solubility. Only the formulations that produced water insoluble materials were selected. For potential applications in biomaterial fields, water insoluble membranes exhibiting good handleability are the most promising.

For a material formulated with M/S of 105 mg/cm$^2$, a solid material was obtained after 68 to 69 hours (see figure 2). After 69 hours, a thin white layer of excess salt forms on the surface of the material as the residual water evaporates. After washing, the layer of salt is rapidly eliminated and a translucent membrane is obtained (see figure 2). An evaporation duration of 7 days was found to be optimal for membranes with M/S of 105 mg/cm$^2$ in order to allow the formation and the compaction of the material.

**First parameter: salt**

First, the solubility of albumin residues prepared without salt was verified, confirming the importance of salt in the formulation of albumin membranes. Then, a thorough screening of many salts at three different concentrations (0.5 M, 1 M and 2 M) was performed. As described previously, the water solubility of the dry residues after 48 hours in distilled water was used to identify the salts that allow membrane formation.

According to Hofmeister series, different ions have different effects on protein stability and solubility. The lyotropic effect is related to the size, the charge density and polarizability of the ions. When working with solutions containing high concentrations of salt and protein, the implication of the interaction between the solvent molecules, the salt's ions and the protein should be considered. During this experiment, 12 salts with various anions and cations couples were tested: KCl, KBr, KI, NaCl, NaBr, NaI, $CaCl_2$, $MgCl_2$, $KC_2H_3O_2$, $NH_4HCO_2$, $K_2CO_3$ and $K_2HPO_4$. The results of this experiment suggest that the formation of albumin membranes is dependent on both the type of salt and its concentration. In the presence of KCl, NaCl or KBr, albumin molecules do not organize into membranes, the dry residue is a mixture of crystallized salt and dry protein which are entirely soluble in water. Additionally, the premature aggregation of albumin in the initial solutions prepared with salts containing divalent anions ($K_2CO_3$ and $K_2HPO_4$) prevents membrane formation. Water insoluble membranes were obtained with 7 of the 12 salts: NaBr, KI, NaI, $CaCl_2$, $MgCl_2$, $KC_2H_3O_2$ and $NH_4HCO_2$. With NaBr, membranes were obtained with all three tested concentration. Although, with the other 6 salts, albumin membranes were obtained only with certain concentrations. The physical aspect and the properties of these membranes (water absorption, initial expansion, handleability) vary considerably depending on the type of salt used and its concentration.

BSA/NaBr 664 membranes (initial salt concentration = 1 M) exhibited good mechanical strength and handleability. These membranes (M/S = 105 mg/cm$^2$) were produced with a relative yield of 87.6 % $\pm$ 4.1 %, their water absorption and initial expansion were estimated at 123.1 % $\pm$ 6.8 % and 121.6 % $\pm$ 4.7 % respectively (n = 8) and their density was 1.29 $\pm$ 0.02 g/cm$^3$. This formulation was selected as a reference for the identification of the parameters for albumin-based membranes formation and for the characterization of these materials.

**Second parameter: salt/albumin molar ratio**

It was shown previously that the salt concentration had an impact on membrane formation. However, it is unclear whether the concentration of salt should be considered as an independent parameter or it should be paired with the concentration of albumin in a given solution. The effect of the initial concentrations of BSA and NaBr on the formation of membranes was assessed by comparing the membranes obtained during two assays: the first assay involved a constant concentration of NaBr and a variable concentration of BSA, therefore a variable molar ratio salt/albumin, while the second assay required both concentrations to be modified at the same time without changing the molar ratio salt/albumin. In the first assay, solutions of 100 mg/mL, 200 mg/mL, 300 mg/mL and 400 mg/mL of BSA were prepared with 1 M NaBr. The molar ratios NaBr/BSA were respectively 664, 332, 221 and 166. Decreasing the molar ratio resulted in a notable decrease of the formulation yield (respectively 86.4 %, 77.2 %, 62.7 % and 0 %) and the obtained membranes vary considerably in terms of visual aspect and water absorption. Furthermore, the residue obtained after the evaporation of the solution with the concentration of albumin of 400 mg/mL (molar ratio salt/albumin = 166) was entirely water soluble. In the second assay, the prepared solutions contained the following concentrations of BSA and NaBr respectively: 100 mg/mL with 1 M, 200 mg/mL with 2 M, 300 mg/mL with 3 M and 400 mg/mL with 4M. The molar ratios NaBr/BSA was 664 in all solutions. Unlike the first assay, all the solutions of the second assay led to membrane formation. The obtained membranes share the same visual aspect and exhibit similar properties. Therefore, the effect of salt concentration on membrane formation cannot be assessed without pairing it with the concentration of albumin in the initial solution. Since the formation of membranes is dependent on the paired effect of both concentrations of salt and albumin, the molar ratio salt/albumin proves to be a more relevant and reliable parameter to evaluate albumin membrane formation.

The next step was to identify the range of molar ratios NaBr/BSA in which membranes can be formed. BSA solutions were prepared with a set concentration of albumin (100 mg/mL) and molar ratios NaBr/BSA from 50 to 2000. These solutions were evaporated and the resulting materials were soaked in water for 48 hours as described earlier. Fully formed membranes were obtained within the range of molar ratios 100 and 3000, particularly within 200 to 2000. For the lowest and the highest molar ratios of this range, the obtained membranes are less robust and more prone to breakage and degradation during handling, but they are however acceptable.

**Influence of albumin surface charge**

Because of the well-established implication of the ionic content of the initial solution on the formulation of albumin membranes, albumin's surface charge should be evaluated to provide a better understanding of the ionic phenomenon leading to membrane formation. Albumin's surface charge is dependent on the pH of the initial solution and its ionic strength.

To evaluate the influence of pH on the formation of membranes, solutions of albumin and NaBr at a molar ratio NaBr/BSA of 664 were prepared at pH values of 4, 5, 6, 7 and 8. With an isoelectric point of 4.7 and an isoionic point of 5.2, BSA has a net negative charge at pH 6, 7 and 8, a net positive charge at pH 4, and is a zwitterion around pH 5. Albumin membranes were obtained at all tested pH values. These membranes shared similar visual aspect and formulation yields but their water absorption and initial expansion were significantly different. The membranes formulated at the highest pH values have higher water absorptions and initial expansions. Therefore, pH seems to have a moderate influence on the formation of membranes. Furthermore, the study of albumin surface charge at pH 6 revealed that by adding salt, the global surface charge of the protein increases due to the interactions between the protein and the cations of the salt. In fact, the measured induced potential increases greatly by increasing the salt concentration before reaching a plateau (see figure 3). The increase of albumin surface charge can result in the reduction of electrostatic repulsions between the molecules and promoting their agglomeration to form albumin membranes.

**Residual salt content**

In anticipation of biological evaluation, the final composition and the residual salt content in the formulated materials should be well characterized. Therefore, to determine the final composition of the BSA/NaBr 664 membranes and quantify the residual NaBr, two complementary methods were used. First, using a BCA assay, albumin was quantified in the rinsing water used for washing the BSA/NaBr 664 membranes. After evaporation of the rinsing solution, the dry residue was weighed and the quantity of NaBr eliminated by the washing process was calculated and compared to the quantity of NaBr used initially to formulate the membranes. The residual NaBr content in the washed BSA/NaBr 664 membrane was estimated at 5.9 % (%wt). However, due to the very low absorption values measured in the rinsing water (concentrations near the lower detection limit of BCA titration), the calculated percentage of residual salt is an overestimation of the actual residual salt in the final membranes. Therefore, to verify these results and target specifically the bromine content, the final composition of BSA/NaBr 664 membranes was directly analysed by microanalysis. Microanalysis was performed directly on the dried membranes and revealed that the bromine content in the tested membranes was 2.36 % (%wt, analyzed surface = 1180 $\mu m^2$, sample thickness = 1 mm, error percentage = 17.74 %). Furthermore, the analysis of the BSA/NaBr membranes by X-ray diffraction (see figure 8) did not detect any traces of crystalline NaBr in the membranes. In conclusion, the NaBr added initially in the solution for the formulation of albumin-based biomaterials is eliminated entirely during the washing process.

**Mechanical properties**

The viscoelastic behaviour of the selected albumin-based biomaterial was studied after its saturation with water. The conservation modulus (G') was found to be higher than the loss modulus (G") (see FIGURE 4). Furthermore, no sol-gel transition was observed. Therefore, within the range of frequency tested, the membranes display a solid-like behaviour. During the compression assay, it was found that the membrane formulated with BSA and NaBr with a molar ratio of 664 has an elastic modulus of 0.7 MPa. Furthermore, tensile tests were carried out on a batch of albumin membranes (n = 6) formulated by the same way. The elastic modulus of the membranes calculated after the traction assay is 0.87 $\pm$ 0.12 MPa. Therefore, both assays give similar results. The maximum stress that caused the breakage of the biomaterial was estimated at 0.19 $\pm$ 0.03 MPa.

Table 1 below shows the comparison between traction and compression results. The membranes used were formulated with BSA (bovine serum albumin) and NaBr at a molar ration NaBr/BSA of 664 under the selected conditions (evaporation at 37 °C and pH = 6). The tests were performed on hydrated biomaterials.

Table 1:

|  | Traction assay | Compression assay |
| --- | --- | --- |
| Initial thickness (mm) | 1.74 $\pm$ 0.08 mm | 1.7 mm |
| Strain to rupture (%) | 26.2 $\pm$ 4.7 % | No rupture |
| Maximal stress (MPa) | 0.19 $\pm$ 0.03 MPa | > 0.13 MPa |
| Maximal force (N) | 3.2 $\pm$ 0.5 N | > 40 N |
| Elastic modulus (MPa) | 0.86 $\pm$ 0.13 MPa | 0.7 MPa |

**Stability in aqueous solutions**

It is important for a biomaterial to be used in contact with biological fluids to have a good stability in aqueous media. For applications such as the formulation of implants or scaffolds for tissue regeneration, the biomaterial should be insoluble in water or have a very slow degradation process. Therefore, the stability in aqueous solutions of the membrane BSA/NaBr 664 was tested. The biodegradability of the membrane was also tested in a trypsin solution (see FIGURE 5).

The membranes incubated in contact with the trypsin solution were completely degraded and dissolved in the buffer. As for the membranes incubated for 7 days at 37 °C without the protease, each batch of membranes lost less than 10 % of its initial mass in each dissolution media, with the exception of membranes incubated in the basic solution, which lost 17% of their mass. Furthermore, the formulated albumin-based membranes are insoluble in an aqueous medium and remain intact for more than 7 days of incubation in those media at 37 ° C. In fact, these membranes could be stored up to a month in distilled water without showing any degradation. In addition, these biomaterials show resistance to acidic and basic pH. The mass loss observed in water and ethanol could be explained mostly by the erosion of the membrane caused by frictions against the sides of the tubes during its agitation. Therefore, these albumin-based membranes are very stable in aqueous solutions and are completely biodegradable.

The stability of BSA/NaBr 664 membranes in solutions of urea (2 M, 4 M and 8 M) and 2-mercaptoethanol (0.1 M) was also tested. Urea and 2-mercaptoethanol are well-described denaturants that can induce protein unfolding by breaking hydrogen bonds and disulfide bridges respectively. Even though the tested albumin-based membranes did not break or dissolve, the diameter of the hydrated membranes (Ah) increased significantly, indicating an increase of their initial expansion (E %), as their complex modulus (G*) decreased, indicating a decrease of their elastic modulus (E) (see FIGURE 6). Furthermore, the breakage of hydrogen bonding and disulfide bridges altered the structure of the membranes but did not allow their dissolution.

## Evaluation of the conformation of albumin

### IR analysis.

Fourier transform infrared spectroscopy (FTIR) is a well-established method used to assess the secondary structures of proteins. The infrared spectra of proteins are characterised by a set of absorption regions in the absorption spectrum known respectively as the amide region and the CH region. Information about the secondary structures can be obtained from the spectra primarily from the amide I region (1700-1600 cm-1) and the amide II region (1600-1500 cm-1). The amide I region reflects mainly the C=O stretching vibration of the peptide group, which gives information on the proteins secondary structures. Furthermore, this technique allows the exploration of the secondary structures of proteins contained in highly concentrated solutions and in solid materials and can either be used with hydrated or dry materials. FTIR was used to evaluate the structure of albumin in BSA/NaBr membranes. In this experiment, to prevent the $H_2O$ bands from interfering with the Amid I band located in the same absorption range, $D_2O$ was used to hydrate a readily available BSA/NaBr 664 membrane and to prepare two control solutions: a solution of BSA (100 mg/mL) and a solution of BSA and NaBr (BSA concentration = 100 mg/mL, BSA/NaBr ratio = 1:664) comparable to the one used to formulate the BSA/NaBr 664 membranes.

The analysis of the deconvoluted spectra of the amid I band revealed the existence of multiple subbands that were identified using the data already available in the scientific literature. Six subbands were found in the amide I band of the tested samples: $\alpha$-helix (1655 cm-1), $\beta$-sheets (1612, 1629 and 1678 cm-1), $\beta$-turns (1669 cm-1) and random coils (1643 cm-1) subbands (see figure 7). The percentage of these secondary structures was then calculated (residual RMS error < 0.005). Very similar percentages of $\beta$-sheets, $\beta$-turns, $\alpha$-helix and random coils were obtained for the albumin present in the solutions of BSA and BSA/NaBr 664. Therefore, in the established experimental conditions, NaBr does not alter the secondary structure of albumin. However, in the BSA/NaBr 664 membrane, there seems to be an increase in $\beta$-sheets and $\beta$-turns and a decrease in $\alpha$-helix and random coils (Table 2). The unfolding of proteins is characterized by an increase in random coils which are inorganized secondary structures. In the formulation of the membranes, the albumin seems to some of these inorganized structures in favour of $\beta$-organized structures.

Table 2 below shows the analysis of the percentage of each secondary structure identified in the amid I band of albumin present in a BSA solution (100 mg/mL, in $D_2O$), a BSA/NaBr 664 solution (BSA concentration = 100 mg/mL, in $D_2O$) and a BSA/NaBr 664 membrane (residual RMS error < 0.005). A BSA solution (BSA 100 mg/mL 80 °C $D_2O$) was incubated overnight at 80 °C and was used as a reference for denatured protein.

Table 2:

| | $\alpha$ helix | $\beta$ sheets | $\beta$ turns | Random coil | Intermolecular $\beta$ sheets |
|---|---|---|---|---|---|
| BSA/NaBr 664 solution $D_2O$ | 31.7% | 28.5% | 6.0% | 33.8% | 3.8% |
| BSA/NaBr 664 membrane $D_2O$ | 21.8% | 46.8% | 16.5% | 14.8% | 23.6% |
| BSA 100 mg/mL $D_2O$ | 30.6% | 28.2% | 6.9% | 34.3% | 2.9% |
| BSA 100 mg/mL 80 °C $D_2O$ | 8.8% | 48.8% | 5.7% | 36.7% | 2.6% |

### Crystallography SAXS ICS

The analysis of the scattering curves presented in figure 8 reveals that:

1) There is no crystalline NaBr in the tested biomaterials.
2) The scattering curves of the tested biomaterials are identical regardless of the molar ratio NaBr/BSA used for the preparation of the biomaterials.
3) The distance between the mass centres of BSA molecules is higher in the biomaterials than in the control powder. The maximum of the peak at 0.19 Å-1 in BSA powder is shifted to 0.16 Å-1 in the biomaterials. Therefore, the distance between the mass centres of two neighbouring BSA molecules increases from 33 Å in BSA powder to 40 Å in the biomaterials.

The secondary structures of BSA is preserved in the biomaterials in comparison to the BSA powder. The peak with a maximum at 0.65 Å-1 is characteristic of $\alpha$-helix structures (diameter of $\alpha$-helix: 10 Å). This peak is not altered in the tested biomaterials. The slight change of shape and amplitude of the peak can be linked to a minor modification of the $\alpha$-helix content of BSA in the biomaterial. The peak with a maximum at 1.4 Å$^{-1}$ is characteristic of $\beta$-sheets (strand-to-strand distance of $\beta$-sheets: 4.7 Å). This peak is modified in the scattering curves of the tested biomaterials and of the BSA powder in comparison to those of BSA solutions.

**SEM surface analysis**

In order to study the surface topography of membranes formulated with NaBr (molar ratio NaBr / BSA = 664) and to evaluate their porosity, the membranes were analyzed using SEM. The surface of the membrane seems to be highly rough with many pores and pore-like substructures (A, figure 9). An observation of a slice of this same membrane (cross-section) shows the existence of small pores inside the biomaterial. However, there does not seem as much as one might think by observing the surface (B, figure 9). The surface irregularities are mostly due to the evaporation used for formulation.

**Other albumin proteins**

The formulation procedure developed produced albumin-based membranes using BSA with a good repeatability. Formulations with other albumin proteins were carried out to study the feasibility of these membranes. Two albumins have been selected, human serum albumin (hSA), which has a very similar structure to bovine serum albumin, and ovalbumin (OVA), which has a structure and a molecular weight different from the two other proteins. Interesting membranes were obtained with both hSA and OVA. These have a different morphology than BSA membranes. In addition, they are more prone to hydration, resulting in higher water content and initial swelling. In conclusion, the established formulation process allows the preparation of albumin-based membranes regardless of the origin of the protein. However, only the BSA-based membranes were further characterized in this study due to the lower cost of this protein and its close resemblance to the human one.

Other membrane formulations were tested with different proteins, as y-globulins. The main constraint was the protein solubility in water. In fact, albumin has an unmatched solubility in water allowing the preparation of solutions at very high concentrations. If the protein concentration should be lowered because of the protein's solubility threshold, the volume required to reach the minimal thickness compatible with membrane handling should be increased, prolonging considerably the evaporation processing. Membranes of $\gamma$-globulin (solubility in water at 20 °C $\approx$ 20 mg/mL) were successfully formulated by evaporating a solution of bovine $\gamma$-globulin and NaBr (molar ratio salt/protein = 664, M/S = 35 mg/cm$^2$). Therefore, the formulation procedure developed in this work proves to be promising for the formulation of protein-based membranes.

**Biological assays**

**Cytotoxicity and cell adherence**

The biological properties of BSA/NaBr 664 membranes were studied. Two other interesting membranes were included in these experiments to have comparable references, BSA/NaBr 400 and BSA/CaCl$_2$ 700. First, the cytotoxicity of the membranes leachable components was evaluated by incubating Balbc 3T3 mouse fibroblasts in membrane extracts. For this cell line, the cell viability can be estimated by measuring the metabolic activity. The normalized metabolic activity of the cells cultivated with each extract was then compared to the metabolic activity of untreated cells (positive control). For this experiment, the extracts were diluted to reveal any dose-dependent effect. The statistical analysis of the obtained data concluded in no significant difference between the metabolic activity of the untreated group and the groups treated with various dilutions of the BSA/NaBr membrane extracts (see figure 10). Furthermore, the non-cytotoxicity of albumin and the absence of NaBr in the final membranes as shown previously match the observed non-cytotoxicity of the leachable component of the BSA/NaBr membranes. Then, the direct cytotoxicity was assessed by incubating Balbc 3T3 cells directly with these membranes. Relatively thin membranes (average thickness $\approx$ 0.7 mm) were used in this experiment to allow the follow-up of the interaction between the cells and the material by microscopy (see figure 11). The statistical analysis of the data showed no significant difference between the metabolic activity of the untreated cells and the cells cultivated with the tested membranes (see figure 10). Furthermore, the microscopic examination revealed that the fibroblasts were spreading around as well as above the membranes (see figure 12). To quantify cellular adherence on the membranes, Balbc 3T3 cells were incubated for 24 hours with the membranes. Then, the culture media was eliminated, the membranes were transferred to an empty well and the metabolic activity of the cells on each membrane was measured. A significant difference was observed between the BSA/NaBr 664, the BSA/NaBr 400 and the BSA/CaCl$_2$ 700 groups (p < 0.05). Around 45 % (44.55 % $\pm$ 10.67 %) of the fibroblasts seeded adhered on the BSA/NaBr 664 membranes. The estimated percentage was lower for the BSA/NaBr 400 membranes (27.36 %

$\pm$ 11.22 %) and higher for the BSA/CaCl$_2$ 700 membranes (77.62 % $\pm$ 20.26 %) (see figure 13). Therefore, the initial formulation solution seems to have a significant effect of the interaction of the cells with the membrane despite the complete elimination of the salt during the washing process. In conclusion, the formulated albumin-based biomaterials are non-cytotoxic and are in favour of cell adhesion and colonization.

**Macrophage activation**

The effect of the albumin membranes on macrophage activation was evaluated by measuring nitrite and TNF-$\alpha$ concentration. NO and TNF-$\alpha$ are produced by activated macrophages to initiate and sustain the inflammatory response. Raw macrophages were cultivated with the tested membranes for 24 h. Afterwards, LPS was introduced directly in the wells of the LPS-activated groups to activate the macrophages and the cells were incubated for another 24 h. In comparison with the non-treated group (NT), nitrite production undergoes a slight increase in the presence of the tested membranes ($p < 0.05$). The LPS activation induces a significant increase of the nitrite concentration in the culture media. However, in comparison with the LPS-treated control (T LPS), nitrite production seems to be significantly lower when the macrophages are cultivated with albumin membranes ($p < 0.05$). TNF-$\alpha$ production follows a similar trend for the inactivated groups. Although, there is no significant difference between TNF-$\alpha$ production in BSA/NaBr groups and the T LPS group, unlike the BSA/CaCl$_2$ 700 group that showed a significantly lowered TNF-$\alpha$ production ($p < 0.05$). Therefore, the tested albumin-based membranes do not efficiently induce the inflammatory response by activating macrophages.

**Conclusion**

In these tests, several interesting biomaterial models have been developed with the following salts: NaBr, NaI, KI, CaCl$_2$, MgCl$_2$, potassium acetate and ammonium formate. The properties of these membranes could be modulated by modifying the formulation parameters. Two parameters of importance were identified: the presence of salt and the salt/albumin molar ratio. Also, it is possible to obtain membranes with ternary systems salt1/salt2/albumin or salt/albumin/polymer, which were successfully formulated. These systems make it possible to modulate the properties of the membranes (mechanical and intrinsic properties) and to obtain functionalized biomaterials with new properties. Albumin-based biomaterials formulated by evaporation in the presence of salt form a versatile model whose properties can be modulated to adapt to the requirements of targeted therapeutic applications.

**Claims**

1. Process of preparation of a biomaterial comprising the steps of:

   a) Preparing a solution comprising at least one protein having a solubility in water superior or equal to about 10 mg/ml and at least one salt having solubility in water superior or equal to about 500 mg/ml,
   b) Evaporating the solution obtained in step a) as is, as a foam obtained by foaming the solution obtained in step a), or as a mixture thereof, at a temperature comprised of from 20 to 50°C in atmospheric pressure or at lower temperatures under vacuum or at a pressure lower than atmospheric pressure, until the formation of two non-miscible phases or until obtaining a substantially dry solid,

   thereby obtaining a biomaterial.

2. Process according to claim 1, wherein said at least one salt is selected among NaBr, NaI, KI, CaCl$_2$, MgCl$_2$, KC$_2$H$_3$O$_2$ and NH$_4$HCO$_2$.

3. Process according to claim 1 or 2, wherein said dry solid obtained in step b) is washed until elimination of at least 90%wt of said at least one salt.

4. Process according anyone of claims 1 to 3, wherein said at least one protein is selected among serum protein such as albumin, or globulin.

5. Process according to claim 4, wherein said albumin is selected among human serum albumin, bovine serum albumin, porcine serum albumin, ovalbumin, vegetal albumin, or recombinant albumin.

6. Process according to anyone of the preceding claims, wherein at least one additive is added during step a) and/or step b).

7. Process according to claim 6, wherein said at least one additive is selected among polymers, notably non charged, positively charged, negatively charged and zwitterionic polymers, non-ionic amino acids and particles.

8. Process according to anyone of the preceding claims, wherein said at least one protein and said at least one salt are in a molar ratio comprised between 100 and 3000.

9. Biomaterial obtainable by the process as defined in anyone of the preceding claims.

10. Biomaterial according to claim 9, **characterized in that** the percentage of secondary structures of said protein is at least the same as in corresponding native protein.

11. Biomaterial according to anyone of claims 9 to 10, **characterized in that** it is associated with at least one active ingredient.

12. Biomaterial according to claim 9 to 11, wherein said at least one active ingredient is selected among anti-cancer substances, anti-inflammatory agents, immunosuppressants, immunomodulators, modulators of cell-extracellular matrix interaction including cell growth inhibitors, anticoagulants, antithrombotic agents, enzyme inhibitors, analgesic, antiproliferative agents, antimycotic substances, cytostatic substances, growth factors, enzymes, hormones, steroids, non-steroidal substances, and anti-histamines.

13. Use of a biomaterial according to anyone of claims 9 to 12, as a support for in vitro tissue engineering and/or for in vitro cell culture and in vitro expansion and/or as an implantable medical device.

14. Biomaterial as defined in anyone of claims 9 to 12, for use as a drug.

15. Biomaterial for use according to claim 9 to 12, to replace defective tissues in a subject in need thereof, or in a drug release system.

Figure 1

Figure 2

Figure 3

Figure 4

Figure 5

Figure 6

Figure 7

(a) BSA (dry powder)
(b) BSA (solution 1, $H_2O$: 24.21 %wt)
(c) BSA (solution 2, $H_2O$: 39.09 %wt)
(d) NaBr (dry powder)

(e) BSA/NaBr 400 (membrane)
(f) BSA/NaBr 664 (membrane)
(g) BSA/NaBr 700 (membrane)
(h) BSA/NaBr 1400 (membrane)

Figure 8

A    B

Figure 9

Figure 10

Figure 11

Figure 12

Figure 13

Figure 14

Europäisches
Patentamt

European
Patent Office

Office européen
des brevets

# EUROPEAN SEARCH REPORT

Application Number

EP 19 30 6387

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WANG X ET AL: "Silk nanospheres and microspheres from silk/pva blend films for drug delivery", BIOMATERIALS, ELSEVIER, AMSTERDAM, NL, vol. 31, no. 6, 1 February 2010 (2010-02-01), pages 1025-1035, XP026814129, ISSN: 0142-9612 [retrieved on 2009-11-27] * Abstract, sections 2.1-2.4, 2.10 * | 1,3-7, 9-11, 13-15 | INV. A61L27/22 A61L27/54 A61L27/56 C12N5/00 |
| X | GENNADIY GLIBITSKIY ET AL: "Textures on the surface of BSA films with different concentrations of sodium halides and water state in solution", NANOSCALE RESEARCH LETTERS, vol. 10, no. 1, 28 March 2015 (2015-03-28), XP055683796, US ISSN: 1931-7573, DOI: 10.1186/s11671-015-0860-0 * Abstract; sections "Methods", "Results and discussion" and "Conclusions"; Figures 4 and 6 * | 1,2,4,5, 8-10 | |
| X | US 2015/073551 A1 (UEHLIN ANDREW [US]) 12 March 2015 (2015-03-12) * example 3; claims 1-16 * | 1,2,6-15 | TECHNICAL FIELDS SEARCHED (IPC) A61L C12N |

The present search report has been drawn up for all claims

| Place of search | Date of completion of the search | Examiner |
|---|---|---|
| The Hague | 8 April 2020 | Cadamuro, Sergio |

EPO FORM 1503 03.82 (P04C01)

## ANNEX TO THE EUROPEAN SEARCH REPORT
## ON EUROPEAN PATENT APPLICATION NO.

EP 19 30 6387

This annex lists the patent family members relating to the patent documents cited in the above-mentioned European search report.
The members are as contained in the European Patent Office EDP file on
The European Patent Office is in no way liable for these particulars which are merely given for the purpose of information.

08-04-2020

| Patent document cited in search report | Publication date | Patent family member(s) | Publication date |
|---|---|---|---|
| US 2015073551 A1 | 12-03-2015 | NONE | |

For more details about this annex : see Official Journal of the European Patent Office, No. 12/82